# EUROPEAN PATENT APPLICATION

(11) **EP 4 000 607 A1**
(43) Date of publication of application: **25.05.2022**
(21) Application number: 20840902.9
(22) Date of filing: 11.06.2020
(51) Int. Cl.: A61K 31/14, A61K 31/407, A61P 25/08, A61P 29/00

(54) **SYNERGISTIC COMBINATION OF S-KETOROLAC AND PREGABALIN IN A PHARMACEUTICAL COMPOSITION FOR THE TREATMENT OF NEUROPATHIC PAIN**

(30) Priority: 16.07.2019 MX 2019008467
(71) Applicant: Amézcua Amézcua, Federico, Guadalajara, Jalisco, 44898 (MX); Amézcua Amézcua, Carlos, Guadalajara, Jalisco, 44898 (MX)
(72) Inventor: GARCIA ARMENTA, Patricia del Carmen, RESIDENCIAL ESENCIA CIUDAD DE MEXICO, 45019 (MX); CHAVEZ GARCÍA, Jose Alonso, COL. ESMERALDA CIUDAD DE MEXICO, 14140 (MX)
(74) Representative: Ungria López, Javier
(86) International application number: PCT/MX2020/050011
(87) International publication number: WO 2021/010812

(57) **Abstract**

The present invention relates to the synergistic combination of a non-steroidal anti-inflammatory analgaesic, such as ketorolac or its isomer S-ketorolac or pharmaceutically acceptable salts thereof, and an analogue agent of the neurotransmitter gamma aminobutyric (GABA) from the neuromodulator group, such as the active ingredient pregabalin or pharmaceutically acceptable salt of same, wherein said combination is useful for formulation in a pharmaceutical combination for oral administration, suitable for the treatment of neuropathic pain in patients with recurrent neuropathic pain or in post-surgical patients, as well as in analgesic and anti-inflammatory therapy.

## Description

### FIELD OF THE INVENTION

The present invention is related to the technical field of the pharmaceutical industry, and its object is to provide a pharmaceutical composition comprising the synergic pharmaceutical combination of a non-steroidal anti-inflammatory drug (NSAID), constituted by the active ingredient ketorolac or its racemic forms or pharmaceutically acceptable salts thereof, and a gamma-aminobutyric acid (GABA) neurotransmitter analog agent from the group of neuromodulators, such as the active principle pregabalin or pharmaceutically acceptable salts thereof, as well as pharmaceutically acceptable vehicles, excipients or adjuvants, formulated as oral administration pharmaceutical forms, such as tablets, capsules or pellets. Said combination is indicated for the control and treatment of neuropathic pain.

The combination of the aforementioned active principles provides a greater therapeutic effect if said active principles are jointly administered as a single dosing unit instead of being administered separately, providing benefits such as a lower administered dose, greater therapeutic effect and less adverse effects.

### BACKGROUND OF THE INVENTION

Pain is an unpleasant sensory and emotional experience and as such is personal. Although it cannot be transferred, it can be communicated. This experience is unique, unrepeatable, and transcendent, encompassing neurophysiological, psychological, behavioral, and cultural mechanisms. Thusly defined, pain is a multidimensional, psychophysical, and sociocultural problem with far-reaching implications that transcend patients, their families and even their societies.

A type of pain is neuropathic pain, affecting millions of persons globally, characterized by the perception of abnormal painful sensations, known as allodynia (painful response to an innocuous stimulus) and hyperalgesia (exaggerated painful response to a slightly noxious stimulus). Given its diverse etiology and the kind of discomfort caused by this type of pain, most analgesic compounds generate an unevenly efficacious response to neuropathic pain. Presently, some studies have shown evidence of the usefulness of anticonvulsants, antidepressive and opioid compounds as first choice drugs for the treatment of neuropathic pain, although the usefulness of these drugs could be limited by an unsatisfactory efficacy and by adverse effects that could cause other malaises than the condition subject to treatment. Thus, some associations containing two different analgesics or an analgesic and other classes of pharmaceutical adjuvants have been proposed, in an attempt to deliver a better analgesic effect and/or to reduce undesirable effects such as adverse reactions or negative secondary effects.

Gabapentin (Gbp) or pregabalin (Pgb) or pharmaceutically acceptable salts thereof are currently employed for the clinical management of neuropathic pain. However, whenever pain relief is unsatisfactory, experts have recommended to add a second analgesic agent or adjuvant for accomplishing the treatment.

The potential benefits of combining analgesics, or analgesics with adjuvants, have been well documented in the treatment of several types of pain, including neuropathic pain. A combined therapy can increase the analgesic efficacy, extend the analgesic spectrum, decrease the doses of analgesics or compounds, and diminish secondary or adverse effects. However, it should be noted that some analgesics combinations, or analgesics combined with other agents may not be useful in the treatment of pain, since the analgesic effect of such interactions may be less than the expected effect or compared to drugs taken alone that could deliver a greater effect than the combination.

Some preclinical studies have shown the antinociceptive efficacy of opioids, gabapentin and some NSAIDS administered alone for treating neuropathic pain induced by chronic constriction injury (CCI) of the sciatic nerve in rats, although the effects produced by the combination of pregabalin and active enantiomers of analgesics such as S-ketorolac have not been determined in neuropathic pain caused by CCI. Accordingly, the present investigation determined and assessed the antinociceptive (antiallodynic and antihyperalgesic) effects of Pgb and S-ketorolac given alone or combined, in order to determine their individual effects and the synergic interaction of these drugs in the chronic constriction injury of the sciatic nerve in a rat model, using allodynia and hyperalgesia tests; and for determining the possible adverse effects that could be caused by this drug combination, such as constipation and effects on motor coordination. Also, for modifying lethal doses, therapeutic indexes, and safety windows in the chronic constriction injury of the sciatic nerve in a rat model.

The Bennett and Xie experimental model has been widely used in the study of neuropathic pain and its treatment, since it mimics many pathophysiological properties of neuropathic pain in humans. It is based on the unilateral ligation of the sciatic nerve, causing a chronic constriction injury (CCI lesion). This model has a proven sensitivity to several clinically used drugs for treating neuropathic pain, and a high degree of similitude to other neuropathic pain models in terms of allodynia and hyperalgesia caused by mechanical and thermal stimuli, and for widely used parameters in preclinical pharmacology and the assessment of neuropathic pain.

In Mexico, more than a million persons suffer from neuropathic pain, and in most cases the incidence of pain will last for the remaining years of the patient's life.

Since pain is the main manifestation of these diseases, it is very likely that the patient will self-medicate with commonly used analgesics and both steroidal and non-steroidal anti-inflammatory drugs before seeking for specialized health care. There may be complications if the primary care physician misdiagnoses the disease, or if the referral to a specialist is inadequate. A delayed treatment will most likely worsen the patient's functional prognosis, resulting in high rates of temporary incapacity and disability, high costs of health care loss of productivity costs, quality of life's impairment and even premature death.

Thus, a successful strategy is to combine available and effective active ingredients, such as monodrugs, allowing their mechanisms of action to complement each other, to improve their clinical safety and to obtain a better therapeutic benefit.

The group of nonsteroidal anti-inflammatory drugs is one of the most prescribed globally for the management and treatment of pain-related diseases. NSAIDS have an analgesic effect useful for rheumatic pain, both in inflammatory and degenerative diseases, and are also often used in non-rheumatic diseases such as migraine, dental pain and in other processes involving pain. Moreover, these active ingredients are useful as antipyretics. It should be added that a colon cancer prevention effect of NSAIDS has been proved recently. The use of these drugs is widespread in the general population, even as self-medication, since they are available over the counter, with the potential risk of side-effects.

Some examples of nonsteroidal anti-inflammatory active ingredients are: acetylsalicylic acid, salsalate, diflunisal, phosphasal, lysine acetylsalicylate, phenylbutazone, indometacin, tolmetin, sulindac, acemetacin, diclofenac, aceclofenac, nabumetone, ibuprofen, naproxen, ketoprofen, ketorolac, flurbiprofen, piroxicam, tenoxicam, meloxicam, mefenamic acid, meclofenamate, celecoxib, etoricoxib and lumiracoxib.

From these active ingredients, ketorolac has analgesic, antipyretic and antiinflammatory properties, acting on prostaglandin inhibition.

The chemical name of ketorolac is 5-Benzoyl-2,3-dihydro-1H-pyrrolizine-1-carboxylic acid, and is represented by the following molecule (I):

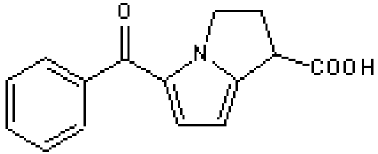

Described for the first time in US Patent No. 4,089,969, with properties for treating pain, inflammation and pyrexia.

The other active ingredient group of gamma-aminobutyric acid (GABA) neurotransmitter analog agents from the group of neuromodulators are often used as antiepileptic drugs and analgesics for neuropathic pain.

Neuromodulators are endogenous substances produced by the metabolism. Without being released by nerve endings nor building-up in them, they act presynaptically, modulating the synthesis and/or release of neurotransmitters. Endogenous neuromodulator molecules are generally known as neuropeptides, since their structures are those of peptides, polypeptides, or proteins. There are also exogenous molecules or neuromodulating drugs, such as gabapentin, pregabalin and carbamazepine, indicated as coadjuvants for the treatment of neuropathic pain.

Neuromodulators can be administered by different routes, especially by oral route. It is very important to know the advantages and limitations of each route. An ill-chosen route may result in more side effects, greater costs and less therapeutic effectiveness.

Structurally, pregabalin is a gamma-aminobutyric acid (GABA) analog, although it does not cause effects on GABA receptors or related structures. It acts by blocking calcium channels, reducing the entrance of calcium to presynaptic nerve endings, and thus decreasing the release of excitatory neurotransmitters (glutamate, noradrenalin and substance P). As a result, it blocks the dispersion of neural excitatory signals.

The chemical name of pregabalin is (3S)-3-(aminomethyl)-5-methylhexanoic acid, represented by the following molecule (II):

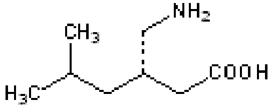

The synthesis of this molecule in racemate forms was first described in an article by R. Andruszkiewicz and R. B. Silverman (1989) and was later protected as active principle in its S form in Mexican patent MX 215885, along with its preparation process and therapeutic use as anticonvulsant, anti-anxiety and antipsychotic.

Pregabalin's mechanism of action is not well understood, although studies involving structurally similar drugs suggest that the presynaptic binding of pregabalin to voltage-gated calcium channels is key for the anticonvulsant and antinociceptive effect observed in animal models. When binding to the voltage-gated calcium channels' alfa2delta subunits in the CNS, pregabalin modulates the release of several excitatory neurotransmitters. Also, pregabalin prevents the migration of alfa2delta subunits from the dorsal root ganglia to the spinal dorsal horn, thus contributing to the inhibition of the release of excitatory neurotransmitters. While pregabalin is a structural derivative of the gamma-aminobutyric acid inhibitory neurotransmitter, it does not directly bind to the GABA or benzodiazepine 8 receptor, i.e., pregabalin fixes itself to auxiliary subunits (proteins a2d) of the voltage-gated calcium channels in the central nervous system (CNS) located in the presynaptic endings of brain and spinal neurons, thus potentially displacing [3H]-gabapentin.

An effective treatment is required for managing neuropathic pain, as well as inflammatory, convulsive, anxiety or psychotic conditions. Said treatment must provide the needed effect for these conditions, with smaller doses than those commonly used, in less time and with fewer adverse effects. Accordingly, the present invention comprises the combination of ketorolac, its active isomers or pharmaceutical salts and pregabalin for the treatment of neuropathic pain, inflammation, convulsions, anxiety or psychosis.

The potential combination of two or more drugs is supported by clinical and experimental data. These combinations have shown analgesic effectiveness, with less adverse effects than the drugs when administered separately. The combination of drugs acting with different pharmaceutical mechanisms of action can provide potential benefits such as:
- Increasing the analgesic efficacy through a synergic or additive interaction.
- Extend the analgesic spectrum: opioids decrease the selectivity to stimuli unrelated to pain, and adjuvant drugs diminish the selectivity to stimuli induced by pain.

Hence, the combination allows to:
- Reduce drug doses.
- Reduce adverse effects.

Given the number of pathways involved in the perception of pain, the World Health Organization and the American Pain Society have recommended therapeutic combinations. When reducing doses, patients have a better tolerance to analgesics.

When two drugs are jointly administered, their effects can be:
a) Additive, i.e., the sum of the effects delivered by the drugs when administered separately.
b) Subadditive, also known as antagonistic; an effect lower than the sum of the drugs when administered separately.
c) Synergic or supra-additive; an effect greater than than the sum of the drugs when administered separately.
d) Potentiation. The result of using an analgesic with a non-analgesic drug, and this combination results in a greater effect than that observed when using only an analgesic.

Evidently, the combination of synergism or potentiation-producing drugs is a more promising treatment of pain. When considering that synergism is usually associated with significantly lower doses and less adverse reactions, the research of drugs administered jointly and resulting in a synergic interaction becomes highly important in pharmacology.

In the state of the art, US Patent 6,720,001 describes a stabilized pharmaceutical oil-in-water emulsion for delivery of a polyfunctional drug, wherein the emulsion has a mean particle diameter of less than about 5 µm and consists essentially of: (a) a therapeutically effective amount of ketorolac, pregabalin or other drugs, and combinations thereof; (b) an aqueous phase; (c) an oil phase consisting of a mixture of structured triglycerides and a polarity modifier effective to facilitate the incorporation of the drug, and (d) an emulsifier suitable for parenteral administration, said composition can be formulated in different pharmaceutical forms; US Patent 9,283,192 describes a press-coated tablet formulation for a delayed, followed by a prolonged release of an active agent comprising: (a) a core comprising the active agent selected from ketorolac, pregabalin, among other drugs, and combinations thereof, together with a wax and optionally one or more fillers; and (b) a delayed release layer surrounding the core and comprising a wax and a low substituted hydroxypropyl cellulose in a ratio of 20:80 to 50:50 w/w; wherein the delayed release layer delays release of the active agent within the core for between 2-8 hours after administration, wherein the low substituted hydroxypropyl cellulose is micronised with a mean particle diameter of 20 µm and has a molecular weight of 115,000 and a hydroxypropyl content of 8%. It also comprises: (c) a top-coating layer comprising an active agent together with one or more excipients, wherein at least 70% of the active agent in the top-coating layer is released within 5-45 minutes following administration; US Patent 9,474,719 describes a variation of US Patent 9,283,192: a delayed release layer surrounding the core and consisting essentially of granules having a size of from 500 µm to 1 mm as measured by sieves; said granules comprising a wax and particles of a low-substituted hydroxypropyl cellulose (L-HPC) in a ratio of 40:60 to 60:40 w/w; wherein the delayed release layer substantially delays release of the active agent within the core for between 3-8 hours after administration of the tablet to a subject and thereafter a pulsed release of the active agent from the core occurs, such that at least 70% of the active agent in the core is released within 5-80 minutes; wherein the L-HPC has a mean particle size of 55 µm, a hydroxypropyl content of 11%, and a molecular weight of 130,000, or a mean particle size of 20 µm, a hydroxypropyl content of 8%, and a molecular weight of 115,000; Patent Applications WO2008115572 y WO2008150324 describe a method of treating pain, fever and/or inflammation in a subject in need of such treatment, said method comprising administering a pharmaceutical composition comprising an ultra-low dose of NSAIDs selected from the group comprising diclofenac, flurbiprofen, ibuprofen, ketoprofen, ketorolac, S(-)ketorolac, naproxene, piroxicam, among other active agents, or pharmaceutically acceptable salts thereof, in racemic form, enatiomeric form or enantiomeric form, and mixtures of materials and characteristics, wherein said composition is administered by the intravenous, intramuscular, subcutaneous, intra-articular, local tissue infiltration or epidural route; wherein said dose in adults is less than about 5 mg if said compound is ketorolac, less than about 2.5 mg if said compound is S(-)-ketorolac, said dosing form also contains one or more analgesics selected from the group consisting of COX-2 selective inhibitors, paracetamol, nitroparacetamol, nitric oxide donors, tramadol, beta adrenergic agonists, alpha-2 agonists, selective prostanoid receptor antagonists, cannabinoid agonists, opioid receptor agonists, NMDA receptor antagonists, gabapentin, pregabalin, gabapentinoids, neuronal nicotinic receptor agonists, calcium channel antagonists, sodium channel blockers, superoxide, costumes apparatus, detoxification apparatus, reinforcement epithelial apparatus. Glycine receptor antagonists and antiepileptics; Patent Application WO2008116165 describes a device for administering a drug to the respiratory system of a patient by aerosol means, wherein the device delivers the drug to the patient in purified air supplied at a positive pressure relative to atmospheric pressure, wherein the device comprises: a purified air generator; a patient interface coupled to the purified air generator; and a medical port coupled to the patient interface and the purified airgenerator, wherein said drugs are selected from the group comprising ketorolac, ketorolac tromethamine, pregabalin, among other drugs, and their combinations; Patent Application PA/a/2006/009919 describes a method for treating or preventing a psychiatric disorder in a subject, comprising the administration of a COX-2 inhibitor selected from ketorolac in combination with a antidepressant agent selected from pregabalin.

The present invention is characterized by providing a composition comprising the combination of ketorolac or its enantiomer S-ketorolac in base form or pharmaceutically acceptable salts thereof and pregabalin or pharmaceutically acceptable salts thereof, in solid oral form, not previously reported in the state of the art. The potential advantage of therapies with this combination is that the analgesic effects may be maximized, while minimizing the incidence of adverse effects.

The use of this drug combination offers an analgesic synergism, thus reducing the required doses and minimizing adverse effects.

### OBJECT OF THE INVENTION

The present invention intends to offer a new therapeutic option for the treatment and management of neuropathic pain capable of reducing symptoms and improving the patients' quality of life. This is accomplished through the strategy of combining ketorolac or its enantiomer S-ketorolac in base form or its pharmaceutically acceptable salt thereof with pregabalin or its pharmaceutically acceptable salt thereof in solid oral form; said combination results in a synergic interaction which increases its therapeutic potency and onset of action, while reducing adverse events.

Said combination improves therapy by offering benefits such as greater effectiveness and therapeutic potency when administered, with the aim of accomplishing local therapeutic effects for the treatment and management of pain caused by neuropathic diseases, inflammation and post-surgery, while significantly reducing the likelihood of side effects.

### BRIEF DESCRIPTION OF THE DRAWINGS

**Figure 1****.** Anti-allodynic temporal course of pregabalin with the acetone test (cold allodynia) in rats with chronic constriction injury. Oral administration of doses with logarithmic increases (0.5 logarithmic units) from 0.0316 to 31.6 mg/Kg. Data represent the mean ± SE, n =6.
**Figure 2****.** Global anti-allodynic effect bars as area under the curve of the corresponding temporal course of different doses (0.0316 - 31.6 mg/Kg oral route) of pregabalin in rats with chronic constriction injury in the acetone test (cold allodynia). Bars represent the mean ± SE, n =6.
**Figure 3****.** Dose-response curve (DRC) of pregabalin plotted as AUCs of the corresponding temporal courses (TCs) versus administered doses of the drug. Graph points are expressed as mean ± standard error.
**Figure 4****.** Anti-allodynic temporal course of S-ketorolac (S-Ket) with the cold allodynia acetone test (cold allodynia) in rats with chronic constriction injury. Oral administration of doses with logarithmic increases (0.5 logarithmic units) from 0.0316 to 100 mg/Kg. Data represent the mean ± SE, n =6.
**Figure 5****.** Global anti-allodynic effect bars as area under the curve (AUC) of the corresponding temporal course of different doses (0.0316 - 100 mg/Kg oral route) of S-Ket in rats with chronic constriction injury in the acetone test (cold allodynia). Bars represent the mean ± SE, n =6.
**Figure 6****.** Dose-response curve of S-Ket plotted as AUCs of the corresponding TCs of global nociceptive (anti-allodynic) effects versus administered doses of the drug. Graph points are expressed as mean ± standard error.
**Figure 7****.** Comparison of S-Ket and Pgb anti-allodynic efficacy determined in the DRC.
**Figure 8****.** TCs of the combination with the peak anti-allodynic effect, and hence the most effective combination among those analyzed for anti-allodynic effects. Also shown are anti-allodynic effects TCs of drugs given alone in the doses used in the combination. Graph points are expressed as mean ± standard error.
**Figure 9****.** Temporal courses of the S-Ket 0.0316 mg/Kg + Pgb 0.10 mg/Kg combination with the peak potentiation interaction (105%) of anti-allodynic effects, compared to the expected anti-allodynic effect theoretical sum of the drugs administered alone. Also shown are TCs of each drug used in the combination. Graph points are expressed as mean ± standard error.
**Figure 10****.** DRCs of S-Ket y de Pgb in antihyperalgesic pharmacological activity when the drugs were administered orally.
**Figure 11****.** TCs of the combination with the peak antihyperalgesic effect, and hence the most effective combination among those analyzed for antihyperalgesic effects. Also shown are antihyperalgesic effects TCs of drugs given alone in the doses used in the combination. Graph points are expressed as mean ± standard error.
**Figure 12****.** Temporal courses of the S-Ket 1.0 mg/Kg + Pgb 0.316 mg/Kg combination with the peak potentiation interaction (23%) of antihyperalgesic effects, compared to the expected antihyperalgesic effects theoretical sum of the drugs administered alone. Also shown are TCs of each drug used in the combination. Graph points are expressed as mean ± standard error.
**Figure 13****.** Temporal courses of the antihyperalgesic effects of the most effective combination compared with the antihyperalgesic effects delivered by the most effective doses of the drugs used in the combination, administered alone.

### DETAILED DESCRIPTION OF THE INVENTION

The present invention refers to the novel pharmaceutical combination to be formulated and administered in a solid oral pharmaceutical composition, containing at least one non-steroid anti-inflammatory agent such as ketorolac or its S-ketorolac isomer in base form or pharmaceutically acceptable salts thereof, such as its tromethamine salt, and at least a gamma-aminobutyric acid (GABA) neurotransmitter analog agent from the group of neuromodulators, such as the active principle pregabalin or pharmaceutically acceptable salts thereof, wherein said pharmaceutically active ingredients provide synergic effects when administered in order to accomplish local therapeutic effects for the treatment and management of neuropathic or post-surgical pain, and as an analgesic and anti-inflammatory therapy.

The S-ketorolac and pregabalin synergic formulation for oral administration intends to provide a quick-onset and effective analgesic and anti-inflammatory effect.

The S-ketorolac and pregabalin combination is an effective therapeutic resource for patients suffering from neuropathic or post-surgical pain, with minimal or no adverse events, and a significant local analgesic and anti-inflammatory action.

The use of an S-ketorolac and pregabalin combination such as the one intended to be protected provides a therapeutic additive effect that has a favorable impact on populations.

The present invention has been tested in preclinical assays, and proves that the novel S-ketorolac and pregabalin combination formulated as a solid for oral administration provides a robust synergic therapeutic effect in the treatment of neuropathic pain, and thus the present invention has as its main object the development of a pharmaceutical composition comprising the combination of a non-steroid anti-inflammatory drug such as ketorolac and the gamma-aminobutyric (GABA) neurotransmitter analog agent from the group of neuromodulators, such as pregabalin or pharmaceutically acceptable salts thereof. Said combination is formulated with pharmaceutically acceptable excipients and is indicated for the treatment and management of neuropathic and post-surgical pain.

Presently, an alternative to increase the efficacy of an analgesic treatment and to significantly reduce the secondary effects is through the combined administration of two or more active agents, such as the synergic pharmaceutical combination intended to be protected by the present invention.

The present invention intends to offer a new therapeutic option for the treatment and management of neuropathic pain in patients with recurrent diseases and post-surgical pain, capable of reducing symptoms and improving the patients' quality of life.

There is presently no assessment of the effects provided by the S-ketorolac and pregabalin oral combination. Accordingly, the present investigation determined and assessed the antiallodynic and antihyperalgesic effects of the S-ketorolac and pregabalin combination, by means of the Bennett and Xie experimental model, which has been widely used in the study of neuropathic pain and its treatment, since it mimics many pathophysiological properties of neuropathic pain in humans. It is based on the unilateral ligation of the sciatic nerve, causing a CCI lesion. This model has a proven sensitivity to several clinically used drugs for treating neuropathic pain, and a high degree of similitude to other neuropathic pain models in terms of allodynia and hyperalgesia caused by mechanical and thermal stimuli, and for widely used parameters in preclinical pharmacology and the assessment of neuropathic pain.

### Method

### Experiment animals

Male Wistar rats were used in the experiments. The rats had a weight of 120-140 g at the beginning of the experimental phase, and a weight of 160-180 g when administering the drugs. The animals were kept in polycarbonate cages under controlled temperature and light conditions, with 12-hour light/darkness cycles and free access to food and water *ad libitum.* All experiments were performed during the light phase of the light/darkness cycles. The animals were used and handled according to the international guidelines on Ethical Standards for Investigations of Experimental Pain established by the Committee for Research and Ethical Issues of the International Association for the Study of Pain. The number of experiment animals was kept to a minimum (n=6), and at the end of the experimental assessments the animals were sacrificed using carbon dioxide.

Immediately after the rats were anesthetized, their right hind limb sciatic nerves were dissected through an incision, in which the biceps femoris muscle was dissected for locating the most proximal site to the trifurcation of the sciatic nerve, and to apply four loose ligations with silk thread.

Once the surgery was finished, the muscle was sutured with absorbable thread, and the skin was sutured with silk thread. Sham surgery in rats was performed likewise, although without ligating the sciatic nerve. All procedures were performed under aseptic conditions. The degree of hyperalgesia and allodynia in rats subjected to sciatic nerve surgery was determined using the Von Frey test (mechanical hyperalgesia) and the acetone test (cold allodynia).

These assessments were performed one day before surgery; 15 days after surgery a 180-minutes temporal course was measured both for the control (carboxymethyl cellulose, CMC) and the compounds administered by the oral route in the study, whether alone or combined, to prove the presence of hyperalgesia and allodynia.

Von Frey test (mechanic hyperalgesia). Rats were placed over a metallic mesh in a transparent acrylic box and remained in the box at least 10 minutes before the test, in order to allow the rats to adapt to the environment. The response was determined by means of a tactile stimulus on the sole callosities of the right hindlimb with the 15-gram Von Frey filament. Ten stimuli were applied (one stimulus approximately every 3 seconds), and the response percentage was obtained (% response = number of responses/10 X 100). With the 15-gram Von Frey filament, controls (sham, no surgery) showed a certain nociceptive response, and thus the response was considered as hyperalgesia.

The acetone test (cold allodynia) was performed and assessed as follows: Once the Von Frey test was finished, rats were left to rest over the metallic mesh, and after a 5-10 minute period, 0.1 mL of acetone was applied on the sole callosities of the right hindlimb with a syringe with a flexible plastic point from under the mesh. Then, a chronometer recorded the time in seconds in which the animals kept the hindlimb away from the mesh during the first 60 seconds after being exposed to acetone. The response time of the right hindlimb was measured. Three measurements were made (1 measurement every 2 minutes).

In a first stage, the experimental design of the groups and the experiments were performed as follows:
a) Several groups of 6 animals with neuropathic pain (sciatic nerve ligation) were formed. In these animals the presence of hyperalgesia and allodynia was determined, both as a result of the degree of neuropathic pain caused in the animals, before and after being treated with the drugs.
b) A control group called SHAM was formed, with a dissection although without ligating the sciatic nerve. This was done to prove that neuropathic pain was present only if the sciatic nerve was ligated.
c) A control group called CCI-VEH was formed, with ligated sciatic nerves, which received the placebo (CMC) and no pharmacological treatment.

Then the effective drug doses ranges were determined in the animals, for analyzing and determining the preclinical effective doses. For determining the DRCs of pregabalin and S-ketorolac given alone, 0.316, 0.1, 0.316, 1.0, 3.16, 10.0 and 31.6 mg/Kg oral doses of pregabalin alone and 0.0316, 0.1, 0.316, 1.0, 3.16, 31.6 y 100 mg/Kg oral doses of S-ketorolac alone were administered, and the antinociceptive effects were assessed with the allodynia and hyperalgesia tests 30, 60, 90, 120 and 180 minutes post-administration, for obtaining the TCs of the significant results for each drug.

### Results

Regarding the allodynia control groups, the CCI group rats showed a marked allodynia in response to the acetone stimulation on the hindlimb that underwent surgery. This test indicated a significant increase in time spent in licking or raising the limb when the CCI rats were stimulated with acetone, in comparison to the SHAM group. As for the hyperalgesia control groups, rats that were not ligated (SHAM) showed a slight nociceptive response when stimulated with the 15-gram filament. The response was almost zero although not absent, which proves that there is a clear nociception with the 15-gram Von Frey filament and that after the CCI surgery rats showed hyperalgesia. In the CCI-VEH group, rats showed a hyperalgesic response, proving that the vehicle had no antihyperalgesic effect.

Anti-allodynic effects:

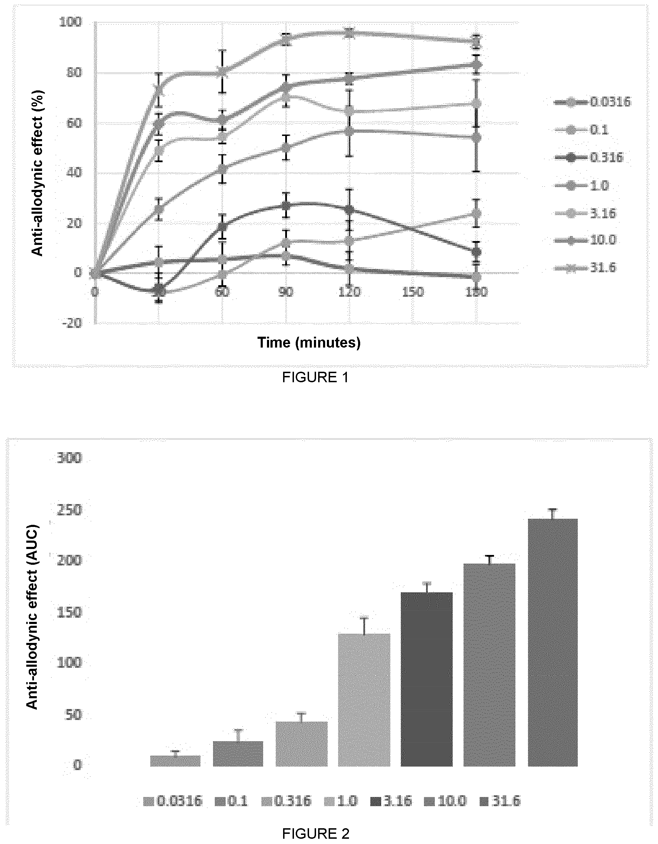

Figure 1 depicts the TCs of the anti-allodynic effects induced by each pregabalin dose, as evaluated in rats with neuropathic pain. The percentages of anti-allodynic responses were assessed for each dose. The X-axis is time in minutes, and assessments were made 0, 30, 60, 90, 120 and 180 minutes after the oral administration of each dose. The Y-axis shows that the animals initially had full allodynia, and that after the administration of pregabalin doses there was a gradual alleviation of allodynia in a dose dependent form, i.e., anti-allodynic effects were recorded. In each experimental point the mean and SE of 6 animals are graphed. There is an evident anti-allodynic effect delivered by pregabalin when the dose is increased. It can also be observed that while a 0.0316 mg/Kg pregabalin dose has almost no anti-allodynic effect, a 31.6 mg/Kg dose induces a significant anti-allodynic effect.
Figure 2 shows the global anti-allodynic effects over a 180-minute time period, depicted the corresponding bars (AUC) of the anti-allodynic effects shown in the TC of Figure 1. Thus, a dose dependent relation is generated.

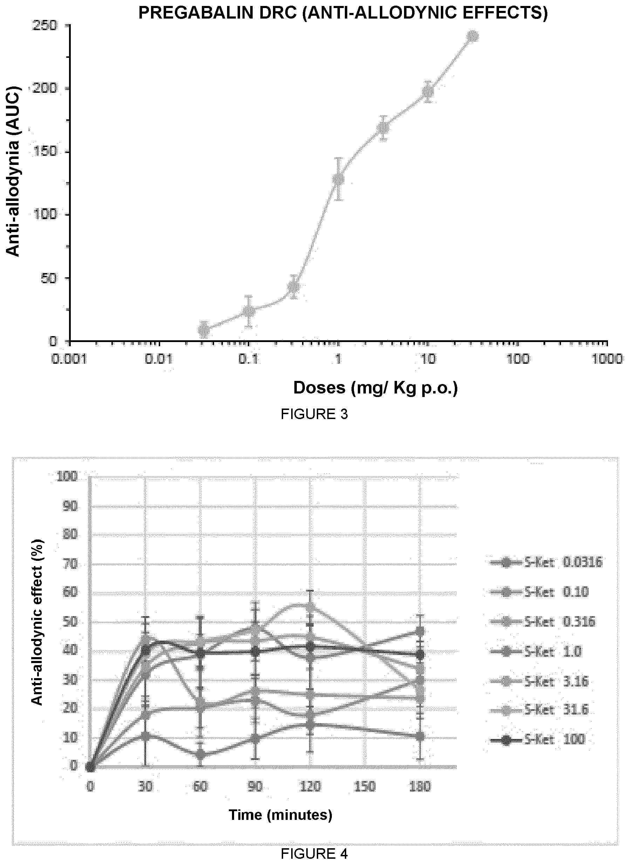

Figure 3 depicts the DRCs of the anti-allodynic effects induced by oral pregabalin in rats with neuropathic pain. Since the TC was 180 minutes or 3 hours, the maximum control AUC possible was established as 300 area units (au). The graph shows the mean ± standard error of 6 animals. A clear dose-dependent relation can be observed in the DRCs.

On the other hand, Figure 4 depicts the TCs of the anti-allodynic effects induced by each S-ketorolac dose, as evaluated in animals with neuropathic pain. The X-axis is time in minutes, and assessments were made 0, 30, 60, 90, 120 and 180 minutes after the oral administration of each dose. The Y-axis shows the level of anti-allodynic effect in animals that initially had full allodynia.

After the administration of S-ketorolac doses there was a gradual alleviation of allodynia in a dose dependent form. In each experimental point the mean and SE of 6 animals are graphed. There is an evident anti-allodynic effect delivered by S-ketorolac when the dose is increased. It can also be observed that while a 0.0316 mg/Kg S-ketorolac dose has almost no anti-allodynic effect, a 1.0 mg/Kg dose induces a maximal anti-allodynic effect.

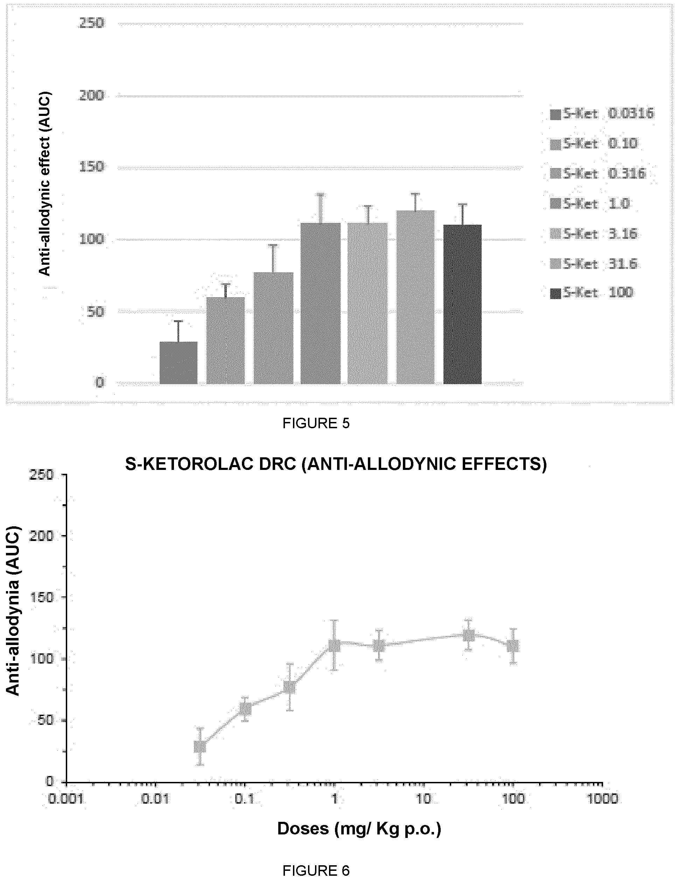

In order to observe the global anti-allodynic effects including the effect duration and maximum effects, the AUC of the respective S-ketorolac dose TCs were calculated. Figure 5 shows the global anti-allodynic effects over a 180-minute time period, depicted as the corresponding bars (AUC) of the anti-allodynic effects shown in the TCs of Figure 4. A dose dependent relation is generated.

Figure 6 depicts the DRCs of the anti-allodynic effects induced by oral S-ketorolac in rats with neuropathic pain. The maximum possible effect, represented by the maximum control AUC has a value of 300 au. The graph shows the mean ± standard error of 6 animals. A dose-dependent relation can be observed in the DRCs. A dose-dependent effect was observed with 0.0316 mg/Kg to 1.0 mg/Kg S-ketorolac doses; maximal efficacy was induced by a 1.0 mg/Kg dose. Although 3 larger doses were assessed, there was no evidence of a greater anti-allodynic efficacy.

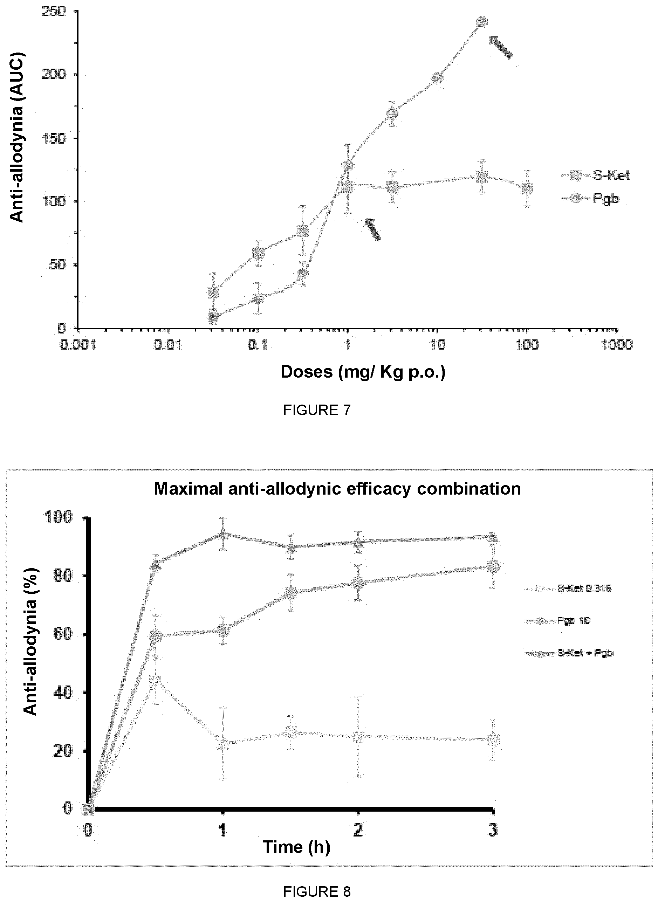

In the case of pregabalin, the maximal efficacy was induced by the 31.6 mg/Kg dose, which reached a maximal anti-allodynic effect of 241.6 ± 9.5 ua, while S-Ket induced its maximal efficacy with a 1.0 mg/Kg dose, delivering an anti-allodynic effect of 111.2 ± 20.3 ua. However, this efficacy was lower than that delivered by pregabalin, as is shown in Figure 7.

Table 1 shows the most efficacious doses of each compound, the maximal efficacy generated and the relative efficacy. It can be concluded that S-ketorolac has only 46% of the anti-allodynic efficacy induced by oral pregabalin.

Hence, pregabalin was more effective than S-ketorolac (both administered orally) for inducing anti-allodynic effects in animals with neuropathic pain caused by CCI.

| Table 1. Anti-allodynic efficacy | | | |
|---|---|---|---|
| Compound | Doses (mg/Kg, po) | Maximal efficacy (AUC: X ± SE) | Relative efficacy |
| Pregabalin | 31.6 | 241.6 ± 9.5 | 1.0 |
| S-ketorolac | 1.0 | 111.2 ± 20.3 | 0.46 |

Once characterized the efficacy of anti-allodynic effects of the 2 compounds when administered alone for neuropathic pain, the interaction and synergism of the anti-allodynic effects induced by the simultaneous administration of these 2 compounds was studied and analyzed, using the "Synergic Interaction Surface" (SIS) methodology.

All combinations to be assessed were designed. It was decided to evaluate combinations with 24 different proportions, to have the fullest possible notion of the interaction of these 2 analgesics, and for obtaining and determining the optimal combinations in terms of efficacy and anti-allodynic potentiation. Since pregabalin delivered the best anti-allodynic efficacy, it was decided to base assessment on 6 doses of the pregabalin DRCs (0.0316, 0.10, 0.316, 1.0, 3.16 and 10 mg/Kg) and to combine them with fixed S-ketorolac doses; 4 different S-ketorolac doses were selected (0.0316, 0.10, 0.32 and 1.0 mg/Kg p.o.).

Table 2 shows all doses used when combining the drugs, the expected effects as theoretical sum (AUC) of the individual effects, the anti-allodynic effects of all combinations as AUCs obtained in the experiments, and the types of interactions or synergisms detected in each case. The 24 different proportions of S-ketorolac + pregabalin combinations to deliver anti-allodynic effects allowed us to detect not only predominantly additive effects, but also some superadditive effects (potentiation).

| Table 2. Type of anti-allodynic effects interaction or synergism of S-ketorolac and pregabalin oral combinations in rats. | | | | |
|---|---|---|---|---|
| a P < 0.05 versus theoretical sum. | | | | |
| b P < 0.01 versus theoretical sum. | | | | |
| S-ketorolac (A) (mg/Kg po) | Pregabalin (B) (mg/Kg po) | Area under the theoretical sum curve (A+B) | Area under the experimental curve (A+B) | Type of interaction |
| 0.0316 | 0.0316 | 47.8 | 105.8b | Potentiation. |
| 0.0316 | 0.100 | 52.3 | 107.6b | Potentiation. |
| 0.100 | 0.100 | 82.9 | 122.6a | Potentiation. |
| 0.316 | 0.316 | 120.2 | 149.8a | Potentiation. |

Regarding the present invention and the experimental model, although 4 combinations generated potentiation interactions, the potentiation and anti-allodynic relief can differ widely and the optimal combination must be detected, since this specific combination can deliver a large potentiation and a significant anti-allodynic relief or high efficacy.

Figure 8 shows the TCs of the combination with the peak anti-allodynic effect, and hence the most effective combination among those analyzed for antinociceptive anti-allodynic effects in the DRCs. Also shown are TCs of each drug used in the combination. Graph points are expressed as mean ± standard error.

In the TC of the optimal efficacy combination (S-ketorolac 0.316 mg/kg + pregabalin 10.0mg/Kg) it can be observed that a good (84.4 ± 3.6%) anti-allodynic effect is promptly achieved (30 minutes post-administration), and that the effect is maintained for 3.5 h until the end of the assessment period (93.6 ± 1.4%) 3 h post-administration. When S-ketorolac 0.316 mg/Kg was administered alone, one of the combination's effective components, a lesser 44.0 ± 7.7% Emax was reached 30 minutes after its administration. This effect decreased significantly until the end of the assessment. On the other hand, pregabalin 10.0 mg/Kg, the other combination effective component, generated its Emax (83.4 ± 3.7%) only 3 h after its administration.

When analyzing the TCs, it may be observed that pregabalin increases its effect over time. However, when adding S-ketorolac to pregabalin, the result is a larger Emax arising in a shorter period (less latency time) after the administration: 0.5 h. That is, with the optimal efficacy combination: 1) The latency until Emax improves (i.e., decreases, which is favorable), 2) The Emax itself improves (i.e., Increases, causing a greater relief), and 3) The anti-allodynic coverage increases (which is useful, since at the end of the evaluation at least one of the components has little anti-allodynic effect, while the combination keeps maintaining and showing a high and very adequate anti-allodynic effect).

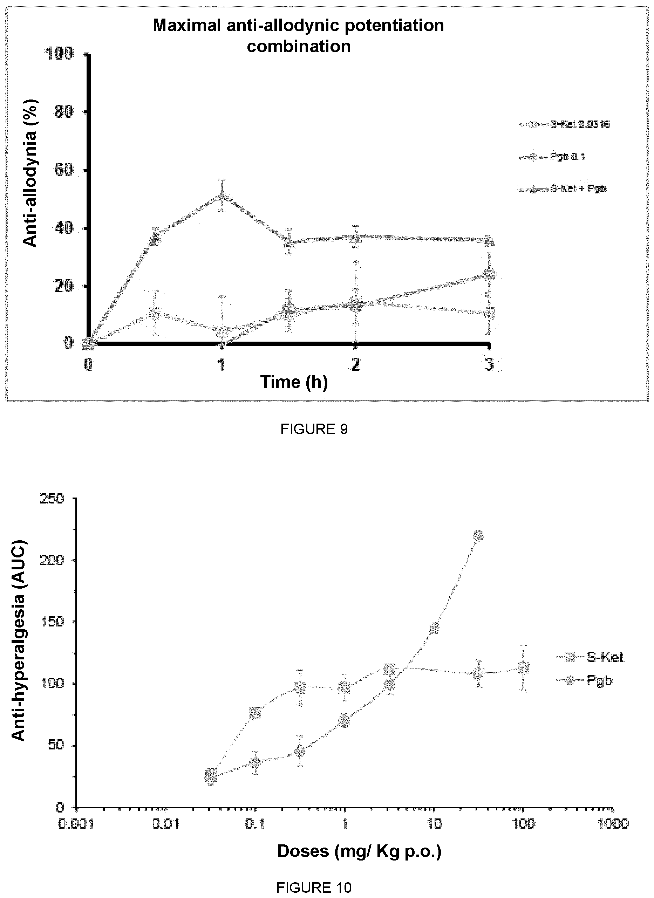

Figure 9 shows the TCs of the combination with the peak potentiation interaction (105%) of antinociceptive anti-allodynic effects, compared to the expected theoretical sum.

As for the antihyperalgesic effects, Figure 10 shows the 2 DRCs obtained for antihyperalgesic effects when administering S-ketorolac and pregabalin alone. In the case of pregabalin, the maximal efficacy was induced by the 31.6 mg/Kg dose, which reached an antihyperalgesic effect of 220.4 ± 12.3 ua, while S-Ket generated its maximal efficacy with a 3.16 mg/Kg dose, delivering an antihyperalgesic effect of 112.5 ± 3.5 ua. However, this efficacy was lower than that delivered by pregabalin.

Table 3 shows the most efficacious antihyperalgesic doses of each compound, the maximal efficacy generated and the relative efficacy. It can be concluded that S-ketorolac reaches only 51% of the antihyperalgesic efficacy delivered by pregabalin. Hence, pregabalin was more effective than S-ketorolac (both administered orally) for inducing antihyperalgesic effects in animals with neuropathic pain caused by CCI.

| Table 3. Antihyperalgesic efficacy | | | |
|---|---|---|---|
| Compound | Doses (mg/Kg, po) | Maximal efficacy (AUC: X ± SE) | Relative efficacy |
| Pregabalin | 31.6 | 220.4 ± 12.3 | 1.0 |
| S-ketorolac | 3.16 | 112.5 ± 3.5 | 0.51 |

Once characterized the antihyperalgesic pharmacological properties of the 2 compounds when administered alone for neuropathic pain, the interaction and synergism of the antihyperalgesic effects induced by the simultaneous administration of these 2 compounds were studied and analyzed, using the "Synergic Interaction Surface" (SIS) methodology.

It was decided to evaluate 24 different combinations, in order to have the fullest possible notion of the interaction of these 2 compounds, and for determining the optimal combinations in terms of efficacy and antihyperalgesic potentiation.

It was decided to base assessments on 6 doses of the pregabalin DRCs (0.0316, 0.1, 0.316, 1.0, 3.16 and 10 mg/Kg) and to combine them with 4 different S-ketorolac doses (0.0316, 0.10, 0.316 and 1.0 mg/Kg p.o.).

Table 4 shows the potentiation combined doses for each drug, the expected effects as theoretical sum (AUC) of the individual effects, the antihyperalgesic effects of all combinations as AUCs obtained in the experiments, and the types of interactions or synergisms detected in each case. The 24 different proportions for combining S-ketorolac with pregabalin to generate antihyperalgesic effects allowed us to detect 3 potentiation combinations.

| Table 4. Type of antihyperalgesic effects interaction or synergism of S-ketorolac and pregabalin oral combinations in rats. | | | | |
|---|---|---|---|---|
| a P < 0.05 versus theoretical sum. | | | | |
| b P < 0.01 versus theoretical sum. | | | | |
| S-ketorolac (A) (mg/Kg po) | Pregabalin (B) (mg/Kg po) | Area under the theoretical sum curve (A+B) | Area under the experimental curve (A+B) | Type of interaction |
| 0.0316 | 0.0316 | 49.6 | 71.7a | Potentiation. |
| 1.00 | 0.316 | 142.5 | 175.0b | Potentiation. |
| 0.100 | 1.000 | 167.5 | 191.7a | Potentiation. |

From the results obtained with the combinations for generating anti-allodynic and antihyperalgesic effects, it is clear that the drug combinations behave differently for the 2 neuropathic pain signs (allodynia and hyperalgesia).

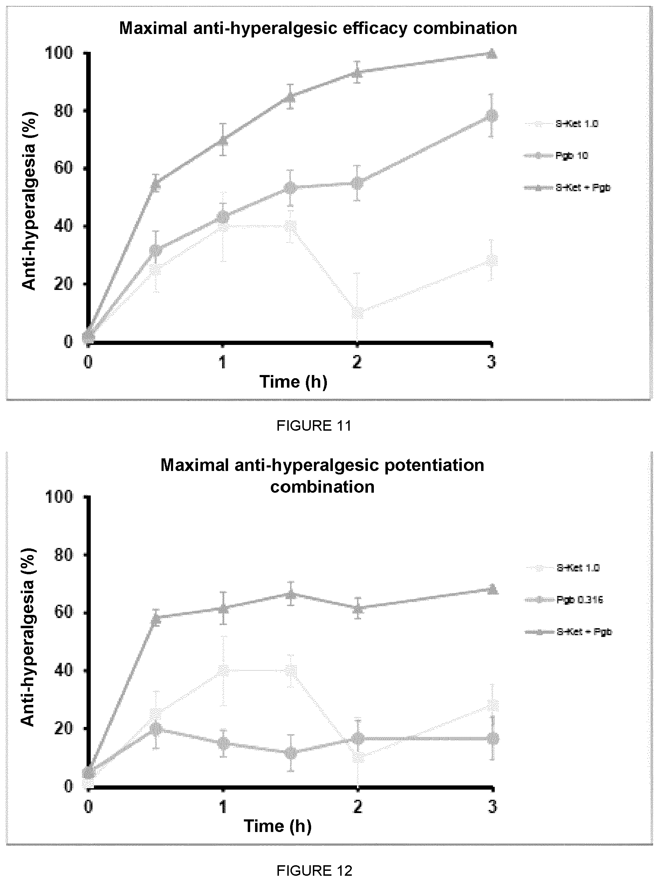

In the TC of the optimal efficacy combination (S-ketorolac 1.0 mg/Kg + pregabalin 10.0 mg/Kg) it may be observed that the antihyperalgesic effect progressively increases over time, finally reaching a 100% effect. When S-ketorolac 1.0 mg/Kg was administered alone, one of the combination's effective components, a lesser 40.0 ± 5.8% Emax was reached 60 minutes after its administration. This effect decreased significantly until the end of the assessment. On the other hand, pregabalin 10.0 mg/Kg, the other combination effective component, generated its Emax (78.3 ± 5.4%) only 3 h after its administration, according to Figure 11.

When analyzing the TCs, it may be observed that pregabalin increases its effect over time. However, when adding S-ketorolac to pregabalin, the result is a larger Emax arising in a shorter period (less latency time) after the administration.

Figure 12 shows the TCs of the combination with supra-additive effect (23%) of antinociceptive antihyperalgesic effects, compared to the expected theoretical sum. Also shown are TCs of each drug used in the combination. Graph points are expressed as mean ± standard error. It may be observed in the figure that S-ketorolac 1.0 mg/Kg delivers little antihyperalgesic effect, while pregabalin 0.316 mg/Kg administered alone induces practically no antihyperalgesic effects over the assessment period. When combining the same doses of these drugs, a 58.3 ± 4.0% Emax was achieved 30 minutes post-administration, an effect maintained for 3.5 additional hours. Hence, the same doses of these drugs combined generated superadditive antihyperalgesic effects 23% larger than the AUC of the individual effects theoretical sum.

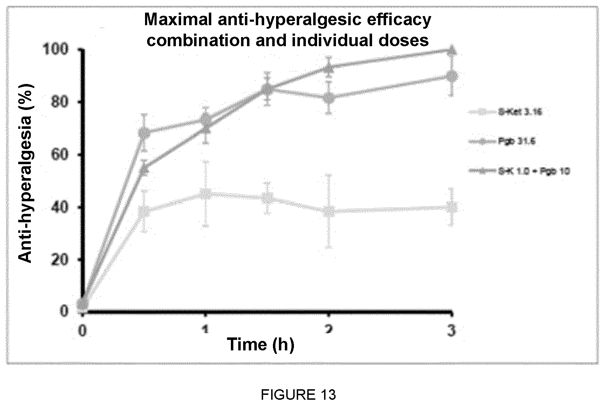

Figure 13 shows TCs comparing the hyperalgesic effects of the maximal efficacy combination (S-ketorolac 1.0 + pregabalin 10) with the antihyperalgesic antinociceptive effects delivered by the highest and most effective doses of the drugs given alone. The antihyperalgesic effects obtained with the combination are better and larger than the effects generated by the most effective S-ketorolac dose (3.16 mg/Kg); the effects of the combination are similar to those obtained with the highest and most effective pregabalin dose (31.6 mg/Kg). However, it should be noted that the combination generated effects similar to those obtained with the maximal pregabalin dose (31.6 mg/Kg), although the combination uses a third of the S-ketorolac dose, and a third of the pregabalin dose.

With the obtained results, and having characterized the anti-allodynic and antihyperalgesic effects, it is feasible that the present invention comprising a S-ketorolac and pregabalin pharmaceutical combination to be formulated and developed as a pharmaceutical composition for oral administration, with smaller doses, greater therapeutic power, and less risk of adverse effects, for treating neuropathic pain.

### EXAMPLES

The following pharmaceutical compositions are described below as non-limiting examples:

### Example 1: Oral administration Compositions for oral administration

| |
|---|
| S-ketorolac or pharmaceutically acceptable salt |
| Pregabalin or pharmaceutically acceptable salt |
| Pharmaceutically acceptable excipient and/or carrier and/or additive |

This invention can be represented in other specific ways without departing from its spirit or essential characteristics, such as parenteral, muscular or intravenous injections; tablets, soft or hard gelatin capsules, micropellets, caplets, powder for reconstitution, with several modified release systems such as controlled release, sustained release or pulsatile release. The described embodiments will be in any aspect considered as illustrative and not as limitations. In said pharmaceutical combination the agent S-ketorolac can be comprised in a strength of approximately 0.001 to approximately 5,000 mg, preferably in a strength of 0.01 to 2,500 mg, and may vary according to necessary adjustments depending on the results of the preclinical study. As for the agent pregabalin, it can be comprised in a strength of approximately 0.001 to approximately 10,000 mg and may vary according to necessary adjustments depending on the results of the preclinical study.

Accordingly, the scope of this invention is defined by the appended claims and not by the above description. Its scope encompasses all modifications within the meaning and range of equivalence of the claims.

In an integral way, this invention provides the following advantages regarding anti-allodynic effects:
1. Both pregabalin and S-ketorolac given orally showed dose-dependent anti-allodynic effects.
2. The S-ketorolac and pregabalin combination predominantly generated additive effects, and also potentiation anti-allodynic affects when combined in precise proportions.
3. The optimal anti-allodynic efficacy combination was S-ketorolac 0.316 mg/Kg + pregabalin 10 mg/Kg (i.e., in a ratio of 1:50, more preferably 1:31.6).
4. The TC of the optimal anti-allodynic efficacy combination shows that S-ketorolac adds its anti-allodynic effect to that of pregabalin, since:
   a) The latency until Emax improves and is reached in less time.
   b) Emax increases and generates a higher effect.
   c) The anti-allodynic coverage increases, with an improved global effect.
5. The combination with the highest potentiation level was S-ketorolac 0.0316 + pregabalin 0.10 mg/Kg (a 105% increase in a 1:5 ratio, more preferably 1:3.16, between the S-ketorolac and pregabalin doses).
6. The TC of the combination with the highest potentiation level shows that adding S-ketorolac to pregabalin:
   a) Improves the latency until Emax
   b) Increases Emax
   c) Increases the anti-allodynic coverage
7. The most effective combination detected (S-ketorolac 0.316 mg/Kg + pregabalin 10 mg/Kg) generated anti-allodynic effects similar to those delivered by the highest and most effective Pgb dose (31.6 mg/Kg), even though the combination used a third of the Pgb dose in the presence of a small amount fo S-Ket (0.316 mg/Kg).
8. This effective combination delivered 124% more anti-allodynic effects than the most effective S-Ket dose (1.0 mg/Kg). Even though the combination used a third of the S-Ket dose.

As for anti-hyperalgesic effects:
1. Both pregabalin and S-ketorolac given orally showed dose-dependent anti-hyperalgesic effects.
2. Pregabalin was more effective than S-ketorolac (pregabalin generated 49% more anti-hyperalgesia than S-ketorolac).
3. The S-ketorolac + pregabalin combination generated preferably additive antihyperalgesic effects, and some potentiation effects.
4. The optimal anti-allodynic efficacy combination was S-ketorolac 1.0 mg/Kg + pregabalin 10 mg/Kg (i.e., in a dose ratio of 1:10).
5. The TC of the optimal antihyperalgesic efficacy combination shows that S-ketorolac adds its antihyperalgesic effects to that of pregabalin:
   a) Improves the latency until Emax
   b) Increases Emax
   c) Increases the anti-allodynic coverage
6. The most effective combination (S-ketorolac 1.0 + pregabalin 10) generated antihyperalgesic effects similar to those delivered by the highest and most effective pregabalin dose (31.6 mg/Kg), even though the combination used a third of the pregabalin dose in the presence of a small S-ketorolac amount (1.0 mg/Kg).
7. This optimally effective combination delivered 100.7% more antihyperalgesic effects than the most effective S-ketorolac dose (3.16 mg/Kg), even though the combination used a third of the S-Ket dose.
8. The optimal potentiation level combination was S-ketorolac 1.0 + pregabalin 0.316mg/Kg (the combination generated 23% more effects than the theoretical sum of individual effects, with S-Ket and Pgb doses in a 3:1 ratio).

Finally:
1. The results prove the usefulness of adding S-ketorolac to pregabalin in the management of neuropathic pain.
2. The S-ketorolac + pregabalin combination delivers excellent additive anti-allodynic results, which may be potentiated.

## Claims

1. A solid pharmaceutical composition for oral administration **characterized in that** it comprises the synergic combination of:
i. an NSAID agent and/or its isomer or its pharmaceutically acceptable salts thereof,
ii. A gamma-aminobutyric acid (GABA) neurotransmitter analog agent from the group of neuromodulators, and/or pharmaceutically acceptable salts thereof
iii. a pharmaceutically acceptable carrier and/or excipient, indicated for the management and treatment of inflammatory diseases, neuropathic pain, or post-surgical pain in mammals; wherein the NSAID is preferably S-ketorolac in base form or pharmaceutically acceptable salt and wherein the gamma-aminobutyric acid (GABA) neurotransmitter analog agent from the group of neuromodulators is preferably pregabalin or pharmaceutically acceptable salts thereof.

2. The composition of claim 1, wherein the agent S-ketorolac is in a concentration of approximately 0.001 to approximately 5,000 mg, preferably a formulation with concentration of approximately 0.01 to approximately 2500 mg.

3. The composition of claim 1, wherein the active agent pregabalin is in a concentration of approximately 0.001 to approximately 10,000 mg.

4. The composition of claim 1, wherein the mammal is a human or animal.
